# EUROPEAN PATENT APPLICATION

(11) **EP 4 008 310 A1**
(43) Date of publication of application: **08.06.2022**
(21) Application number: 20211990.5
(22) Date of filing: 04.12.2020
(51) Int. Cl.: A61K 9/08, A61K 38/00

(54) **FORMULATION FOR THE PROPHYLACTIC TREATMENT OF AN INFECTIOUS DISEASE, IN PARTICULAR OF COVID19**

(71) Applicant: Open Care Pty Ltd trading as Open Care, Chadstone, Victoria 3148 (AU)
(72) Inventor: Bracho Granado, Gustavo Rafael, Black Forest, SA 5035 (AU)
(74) Representative: Schaad, Balass, Menzl & Partner AG

(57) **Abstract**

The present invention relates to a formulation for the prophylactic treatment of an infectious disease comprising two core components, of which a first core component A contains at least one substance S_{A} related to the target disease, and a second core component B contains at least one substance S_{B} for targeting a disease-related homeostatic mechanism that assists core component A in inducing a beneficial environment for developing appropriate protection mechanisms against the disease, wherein substance S_{A} is a biological material from an inactivated pathogen or a part thereof, and substance S_{B} is a synthetic or naturally occurring substance different to substance A. According to the invention, each of the substances S_{A} and S_{B} are contained in the formulation in a concentration lower than 10⁻⁶ g/mL.

## Description

The present invention relates to a formulation for the prophylactic treatment of an infectious disease, and in particular of COVID19 (coronavirus disease 2019), as well as to a process for preparing the formulation.

The global population faces ever-increasing challenges around health and wellbeing. With the rise of internationalized economies and ease of travel, communicable diseases are an increasing risk to everyone, and an increase in the number of global epidemics and pandemics through opportunistic pathogens has to be faced that threaten countless lives and economies. The World Health Organization has prioritised a list of serious pathogens that are most likely to cause severe outbreaks in the near future. Outbreaks threaten the health of humanity and our global economies, whether from naturally emerging and re-emerging diseases or potential bioterrorism-related threats that could occur. The ongoing unprecedented health crisis which threatens a large part of humanity and the world economy, has highlighted how underprepared global health organisations are, how exposed we are globally to health threats, and has made evident the significant limitations of traditional prevention alternatives in protecting the society from rapidly developing pathogens. This emphasizes the need for a different approach to immunisation development and building immunity.

The novel Severe Acute Respiratory Syndrome Coronavirus 2 (SARS-CoV-2) has become the upmost important public health emergency all around the world. The new pandemic of SARS-CoV-2 related disease (COVID19) threatens the health of humanity with increasing numbers of infected people and deaths. Since the epidemic started in December 2019, more than 25 million people have been infected and close to 900 000 deaths have been reported worldwide. Up to date, the only measures available to control the spread are based in avoiding physical contact, personal protective equipment and hygiene as no other prevention tool is yet available despite the accelerated race to develop active immunisation alternatives. Despite the measures adopted by countries that range from promoting individual responsibility, movement restrictions, frontier closures to strict lockdowns and curfews; still the pandemic is out of control and the numbers continuously growing.

The effects are also devastating for states and global economies which has magnified the problem and created huge pressure at all levels.

Particular effort is currently put into the development of an effective vaccine for COVID19. Unfortunately, almost all immunisation strategies, on which the developments of a vaccine are based, are only considering a limited number of mechanisms within the immune system to generate protective immunity. Thus, the pathophysiological outcome of these strategies is often unsatisfying.

In consideration of the drawbacks of the known strategies, the object of the present invention is to provide a formulation for the prophylactic treatment of an infectious disease, said formulation being able to effectively and safely promote beneficial pathophysiology and immune outcomes for individuals who are exposed to a pathogen of the disease, and to SARS-CoV-2 in particular.

The object is solved by the formulation according to claim 1. Preferred features of the invention are defined in the dependent claims.

According to claim 1, the present invention relates to a formulation for the prophylactic treatment of an infectious disease comprising two core components, of which a first core component A contains at least one substance S_{A} related to the target disease, and a second core component B contains at least one substance S_{B} for targeting a disease-related homeostatic mechanism that assists core component A in inducing a beneficial environment for developing appropriate protection mechanisms against the disease. Specifically, substance S_{A} is a biological material from an inactivated pathogen or a part thereof, and substance S_{B} is a synthetic or naturally occurring substance different to substance A.

According to the invention, each of the substances S_{A} and S_{B} are contained in the formulation in a concentration lower than 10⁻⁶ (µg, micrograms) g/mL, and hence in ultra-diluted form (in the following referred to as "ultradilutions" (UD). Preferably, each of the substances S_{A} and S_{B} are contained in the formulation in a concentration lower than 10⁻⁹ g/mL, more particularly lower than 10⁻¹² g/mL.

In contrast to conventional immunisation strategies, which only consider a limited number of mechanisms within the immune system to generate protective immunity, the present invention is based on the finding that better approaches to protective immunity can be developed if the whole system and a broader spectrum of the mechanisms contributing to the immune response are considered.

As will be discussed in the following in detail, the capability of UD (i.e. ultradilutions or substances in ultradiluted form, thus being at a concentration of lower than 10⁻⁶ g/mL, in particular lower than 10⁻⁹ g/mL, more particularly lower than 10⁻¹² g/mL) to provide multiple signals allows to prime the homeostatic mechanisms in a very comprehensive way to provide beneficial pathophysiological outcomes in general, but with an important focus on the immune response.

In recent years the scientific evidence on the effect of ultra-dilutions (UD) on living organisms and the potential mediating mechanisms has grown considerably.

Several studies have demonstrated that highly diluted substances induce measurable effects on cells, plants, animals and humans. Those effects are mediated by the modulation of relevant physiological pathways depending, among other factors, on the ultra-diluted substances used as they are not induced by the dilution vehicle. Considering the nonlinearity of the effect and that all living organisms are very complex systems, the effect of ultra-dilutions should not be viewed as a simple receptor-ligand interaction but rather as a subtle and broader interaction of relevant active mediators present in ultra-diluted substances with several amplification, adaptive and self-regulating mechanisms that comprise the physiology of biological systems.

By combining scientific evidence and empirical deductions from different fields it is possible to provide a theoretical-practical framework that supports the properties of UD and help to explain their effect on biological systems. The main features of this framework are discussed in the following.

The process of diluting any substance in an aqueous solution unavoidably involves the interaction of water molecules with the substance being diluted and such interaction will determine the properties of the UDs. This interaction is driven basically by the properties of the aqueous solution and more importantly by the behaviour of water molecules. Additionally, in the case of agitated dilutions, where every dilution step includes supplying mechanical or kinetic energy to the system, the impact of the additional energy on the thermodynamical properties of the solution should be considered.

It has been demonstrated that silicate nanoparticles (NP) could leach from the glass into the aqueous solution when agitated dilutions (shaking or vortexing) are prepared in glass recipients. Even when it is known that at high concentrations silicate particles could have a biological effect like triggering phagocytosis and cell activation, the concentration of such colloidal nanoparticles is too low in highly diluted solutions in water to account for any effect by their own. Despite that, at low concentrations they could act as core nanostructures where attached molecules could be stabilized. This is apparently the case with substrates and proteins that resulted in increased enzymatic activity and enzyme stability at ultra-low concentrations when silicate nanostructures are present, supposedly due to a direct interaction that maintains the biological activity after being ultra-diluted.

Another set of scientific evidence published in recent years has demonstrated non silica nanoparticles (NPs) derived from the undiluted substance are present in ultra-dilutions and could mediate the biological effect even when the concentration of the substance is far below the threshold for ligand-receptor interaction or toxicity. The presence of these particles has been confirmed by Electron and Inductively Coupled Plasma-Atomic Emission Spectroscopy in dilutions where in theory no molecules of the diluted substance should remain.

This evidence suggests a different solute-solution interaction that does not strictly result in the traditional concept of an exponential decrease of the activity with the decrease in the number of molecules of the solute during the process of serial dilution. Assuming that at high dilutions, derivatives of the diluted substances remain in the form of stable nano particles and that it could exert biological activity, then it is logical to think that the interaction of such nanoparticles with even the simplest biological system is mediated through mechanisms other than the well characterised ligand receptor interaction. The nature of such interaction will then depend on the source and properties of the NP, the solution, the sensing mechanisms and the initial conditions of the system. However, the water molecules in the surrounding vicinity should also play a crucial role as, because of their dipole properties of the molecule, they can self-organise in stable structures depending on the electrical charge distribution on the nano particle. It has been demonstrated in different experiments that several layers of water molecules in the close vicinity of charged surfaces are affected by the electrical charges and associated magnetic field leading to a dynamic and organised orientation that despite the restriction of vibrational and oscillating movement, remain stable thanks to resonance, weak forces molecular forces and hydrogen bonging.

Such organisation appears to be dynamic where there is a continuous replacement of bounded molecules with non-bounded free moving molecules. In that regard, the NPs could serve as a template for the water molecules to self-organise. Regardless of the nature of those NPs, the water molecules that could be dynamically associated with the NPs must play a critical part in mediating the biological effect of aqueous ultra-dilutions. However, the substance derived nanoparticles apparently are not present at the same quantities in all sequential dilutions and it depends on the substance of origin and the dilution method. In addition, they have not been demonstrated to be present in all agitated dilutions, particularly when the substance is being serially ultra-diluted beyond the 10²⁴.

Another line of thinking on the properties of UDs comes from the Physics and Electrodynamics of condensed matter, and more specifically from Quantum Electro Dynamics (QED) when applied to explain the unique and often puzzling properties of liquid water. When the principles used in QED to describe the dynamics of the interactions of atoms and molecules, combined with the effect of classical electromagnetic fields, are applied to model the several thermodynamic anomalies of water, a proposition of two different phases that coexist in liquid water clearly emerge. This model suggests that in liquid water molecules are distributed between a high-density incoherent phase where molecules behave chaotically at ground state and a lower density coherent phase where molecules oscillate in resonance with a large classical magnetic field. The coherent phase comprises a large number of molecules structured together with larger intramolecular spaces and stabilised by the action of the magnetic field in coherent domains (CD), with density close to that of the ice and a predicted size around 100-75 nm (depending on the wave length of the magnetic field).

Within the CD the interactions between molecules are not driven by the well accepted electrostatic forces that mediate ionic bonds, hydrogen bonds and van der Waals forces, but rather they oscillate between a ground and excited state in phase with a magnetic field. The stability of the CD is also maintained by dissipating energy in the form of latent heat or electromagnetic waves with a shifted phase of that of the original field. This also results in less sensitivity to temperature changes of the CD than the incoherent counterpart.

The self-arrangement of molecules in CD implies a more important role of the magnetic field which can be not only stabilizing the cluster but also retained in the liquid by the collection of all CDs. This model where water molecules can be dynamically distributed between the incoherent (gas like) phase and CD nicely explain the thermodynamic behaviour of liquid water, but also its predictions accurately match observed properties ("anomalies").

According to this model, the CD in liquid water can provide the capability to preserve and release the electromagnetic information that is acquired by the influence of low frequency electromagnetic fields. Accordingly, the source of the inducing magnetic field could determine the properties of the CD and the solution.

The structure of most biomolecules defines a specific distribution of charges provided by the presence of delocalised electrons along the molecule. Delocalised electrons could move within certain boundaries along the backbone of the molecule, thus this moving charge generates electromagnetic waves whose properties are essentially defined by the energy of the electron. This is the case of proteins and nucleic acids which are the "task force" that drive almost all bioprocesses. The amino acids that build up proteins provide delocalised electrons to the structure and those electrons are the key in defining the electronic distribution of the whole protein. Considering that the energy of delocalised electrons is different for every amino acid, then the sequence in which they are assembled during protein synthesis (primary structure) will not only define the protein's three dimensional conformation but also the electronic distribution, the spectra of associated magnetic fields and consequently the protein activity. It is well known that the protein function and properties are encoded in its primary structure but in addition the amino acid sequence defines the distribution of delocalised electrons that provide a unique spectrum of electromagnetic frequencies for each protein.

The relevance of the electromagnetic spectrum of proteins and DNA for bioprocess functionality has been put to the fore more importantly by studies of the wave function of electron distribution that lead to the resonance recognition model (RRM) which has been extensively validated with experimental data. The RRM consider the nature of protein interaction to be mainly driven by the electromagnetic frequencies and is based on describing every protein by its frequency spectrum. By comparing the spectra of proteins sharing a common pathway, targets, functional groups or interacting molecules, is possible to identify common characteristic frequencies that are shared among the interacting molecules or pathways (mediating energy transfer between molecules as part of a biological process). Accordingly, it is possible to postulate that each frequency within the unique spectrum of every protein could define a particular function, and that characteristic frequencies are common to interacting molecules. Thus, these complex oscillating periodicities within the distribution of the energy of delocalised electrons not only determine biological functions but also recognition between biomolecules by resonance. This oscillation field of an electromagnetic nature could mediate protein recognition at distance and could also be propagated through the water molecules.

More importantly, the frequencies (waves) generated by delocalised electrons from macromolecules generate a magnetic field that could allow the formation and stabilisation of water CD. In that sense, electromagnetic information from biomolecules could be preserved or stabilised by nanoparticles originated in agitated dilutions but also captured and stored in CDs.

Considering the theoretical-practical framework described previously, using appropriate methods it is possible to prepare an aqueous solution where the main functional signals or properties of the diluted substances could become the preserver and used to stimulate biological systems. The signals could be preserved in agitated dilutions in the form of either stable NPs, clusters of water CD, the physical presence of molecules or a combination depending on the degree of dilution. As it has been suggested, the signals could be mainly of an electromagnetic nature although if the molecules are physically present at relevant concentrations, mechanisms mediated by direct interactions (i.e. ligand-receptor, enzyme-substrate) could be also be activated. However, in similar way that molecules can serve as a template for generating NP and the oscillation of their delocalised electrons could provide magnetic fields for stabilising CD, they themselves can be the templates for de novo generation. The process of ultra-dilution leads to a progressive decrease in the number of molecules of the diluted substance physically present in the solution, but derived NP and water CD can persist as they can be carried and replicated from one dilution to the next. Regardless, those signals carry and provide relevant information that can be sensed by a living system where specific mechanisms are triggered (depending on the diluted substance).

In addition, the action of carrying serial agitated dilutions from the original solution can provide a method for selective filtering out of those signals with a lower intensity or that are less represented while more intense or represented signals would have more likelihood of being preserved. By this method, a refinement of the signals present in the solution is achieved as less relevant signals will be eliminated by diluting. This process is based on the theory that each substance provides several pieces of information with different intensities and frequencies as suggested for the electromagnetic spectra for proteins and DNA, and that the solution is homogeneous so all components would have the same probability of being distributed within the solution as a function of their individual quantities. This highlights the importance of performing vigorous agitation to ensure homogeneity of the solution. On the other hand, it is suggested that NP and water CD can aggregate to form clusters depending on their properties. Thus, the process of agitation helps to disrupt those clusters favouring the process of replication and filtering. In this regard, the force and energy supplied during the agitation is relevant as it should be of enough strength to ensure homogenisation.

Homeostasis can be defined as the tendency toward a relatively stable equilibrium between interdependent elements, especially as maintained by physiological processes. Living organisms have evolved as a complex and sophisticated set of physiological mechanisms to keep the homeostasis and adapt to an environment that is constantly challenging their survival. The homeostatic mechanisms are characterised by their redundancy, bioplasticity and capacity for systemic self-organization which allow all living organisms to behave as a complex adaptive system. When the balance of the system is affected by any challenge or stressor, several interconnected regulatory mechanisms are automatically activated to restore the equilibrium state. The redundancy and interconnectivity of those mechanisms enable several amplification steps and ramifications in a cascade of reactions where all the components function in a highly cooperative manner. Depending on the nature of the stressor, the outcomes of the mechanisms to restore the homeostasis can result in temporary or permanent macro adaptations like the appearance of observable reactions (symptoms) and changes in morphology and behaviour. However, a very large number of underlying reactions take place at systemic, micro and molecular levels which are more difficult to observe and measure but are needed for homeostasis. In fact, the macro effects can be considered as the visible expression of the cooperative action of the underlying mechanisms.

Considering the given living organisms as a complex adaptive system any reaction to a distress should be considered as the expression of those mechanisms regardless of the level at which they are occurring. Additionally, every mechanism in living organisms has a regulatory counterpart that controls the activation pathways to ensure that they don't remain activated longer than needed and the whole system comes back to normal. Those regulatory mechanisms can be considered as well as part of the homeostasis. However, if the dis-balance is strong enough or remains for a long time without being restored, then the regulatory pathways can be overrun, and it can result in an overreaction.

For instance, the symptoms present in a disease, and in COVID19 in particular, can be interpreted as the observable expression of numberless and highly interconnected homeostatic mechanisms at systemic, cellular and molecular levels that have been activated towards recovery. Consequently, the spectrum and severity of the symptoms will depend on the type, intensity, amplification (deregulation) and number of underlying mechanisms that are activated, which in turn depends on the nature and intensity of the stressor.

In the case of infectious diseases such as COVD-19 where the stressor (i.e. infectious pathogen) can replicate itself and remain active for long time, the recovery becomes a competition between the homeostatic mechanisms attempting to eliminate the pathogen and restore homeostasis, and the pathogenic mechanisms of the infection. Unfortunately, many (if not all) pathogens have developed sophisticated ways to divert the homeostatic mechanisms toward ineffective pathways in order to sustain the infection and increase survival. In that scenario, if appropriate mechanisms within the host are not activated with the appropriate intensity and at proper time, then it can result in an over or miss reaction of the system with no beneficial outcomes, and that would lead to severe symptoms and progression of the disease.

The main actor for maintaining the homeostasis in infectious diseases is the immune system. Thus, the immune response can be considered as a homeostatic system with the capability to create and retain memory of the more effective mechanisms for a quicker activation in case of a new contact with the infectious pathogen. The capacity of the immune system to generate memory is an exceptional adaptive feature that constitutes the basis for most immunisation strategies to induce protection. However, since the immune system belongs in a complex adaptive system which the whole body is, the immune response and memory should be regarded as the result of the interactions of multiple underlying mechanisms even outside the immune system, that cooperate to develop immunity.

The immune system is part of body homeostasis intrinsically interconnected with many other systems in such a close relationship that recently has led to the more general concept of the neuro-endocrine-immune system.

The immune system itself, comprises organs, circulating and tissue-resident cells, proteins and other biomolecules structured in a very interconnected, fine-tuned and regulated network. It has evolved to identify and fight dangerous pathogens through several defence mechanisms, but at the same time the immune system has strong regulatory pathways in place that keep it tolerant against non-harmful threats like its own cells and non-dangerous or beneficial microorganisms.

The immune response gets activated commonly after encountering infectious agents that deliver specific danger signals (activation signals) which are strong enough to overcome the regulatory mechanisms. It is generally accepted that the primary source of those danger signals comes from structures and molecules known as Pathogen Associated Molecular Patterns (PAMPs) or more general Danger/Damage Associated Molecular Patterns (DAMPs). The DAMPs are generally recognised by cells of the Innate Immune System (IIS) such as macrophages, neutrophils, tissue-resident dendritic cells and basophils (among others) that are well distributed in all tissues of the body. Those cells are continuously monitoring the surrounding environment for dangerous signals and constitute the component that responds almost immediately after the insult (exposure to pathogens as an example).

In order to respond, these immune cells are equipped with several receptors from a family known as Pattern Recognition Receptors (PRRs) that specifically recognise different DMAPs. As first responders, the cells of the IIS use several nonspecific mechanisms aimed at containing and eliminating the insult; but more importantly, they gather key information and set the conditions for the later recruitment of cells from the Adaptive Immune System (AIS) and their highly specific and more effective defence mechanisms. Despite the recognition of DAMPs by the PRRs which are the main signal for the IIS, the environment (context) in which the infectious agents are encountered has profound implications for the following steps towards the induction of the immune response. The context where infectious pathogens are initially detected not only depends on the pathogenesis of the pathogen but also is determined by the status of surrounding tissue including cells and the intercellular compartment. Commonly this environment is provided by cells of the epithelial surfaces and subjacent tissues (i.e. the skin, mucosal surfaces including the gastro-intestinal urinary track, vagina and respiratory epithelium and vascular endothelium). In turn, epithelial tissues are influenced not only by any effect from the pathogen itself but also by multiple factors involving the nervous system, hormones (endocrine system), metabolic status, immune system mediators and electrodynamic integrity among others. Thus, the environment where the components of the immune system are being activated is multifactorial and mainly dependent on the initial conditions where the pathogen is only one of the factors.

Depending on the stimulus and the cellular and biochemical environment, the initial activation of the IIS triggers changes in gene expression, modification of protein composition on the cell surface, release of several biochemical messengers, cell differentiation and migration just to mention some of the more important responses. These changes are the initial events that start an amplification cascade that induce the recruitment of other cells to the infection site, modifications in the blood vessels and blood circulation, inflammation, adjustments in the cell cycle and metabolisms, changes in cell migration patterns, proliferation and an explosion of secreted biomolecules involved in both defence against the pathogen and signals for other cells and body systems. This becomes an amplification feedback that dramatically changes the environment but that is intrinsically related with its very first steps of activation. All these changes are closely monitored by the immune system, particularly by components of the IIS like dendritic cells, that gather all this information and store it within a complex network of cellular messages to be provided to the AIS.

The information is provided to the AIS by a synergistic combination of soluble factors and surface proteins (expressed on the surface of cells from the IIS) that provide signals to activate putative receptors on B and T cells. After appropriate activation, B and T cells develop different defence antibodies and cell mediated defence mechanisms, which are much more efficient and constitute the final stage towards eliminating the threat. The AIS has the capacity to generate memory by keeping clones of B and T cells that were effectively activated. The combination of memory cells (B & T cells) and the defence mechanisms they exert is what confers long term immunity.

Among the adaptive defence mechanisms, the production of specific antibodies is the most generally accepted indicator of protection; this is why measuring specific IgG levels in sera is often used as a predictor of immunity. The antibodies belong to the immunoglobulin superfamily of proteins which is composed of many classes: IgM, IgG, IgA, IgE and IgD. The IgG is the most abundant class in sera, and it can be further divided into four main subclasses: IgG1, IgG2, IgG3 and IgG4 (in humans). All those classes and subclasses have different properties and functions that fulfil different roles in the immune response. They have major differences in antigen affinity, cell receptors, complement binding capabilities, half-life, levels in sera, placental transport, secretion levels in mucosal surfaces (levels in mucosal secretions like saliva, vaginal fluids, lungs secretions and intestinal milieu) and time frames when they are produced. The role of antibodies in the immune response depends to a large extent on the pathophysiology of the pathogen. Still, they would only be protective if the appropriate kind of antibody is produced at the right place and time.

However, antibody production could be misleading as a measure of protection in many circumstances such as cancer or intracellular pathogens (e.g viruses) where eliminating the cancer or virus bearing cells is much more important, and antibodies are not effective at doing this. In those cases, cell-mediated mechanisms become more important than the antibody-mediated one in order to provide robust protection. With the right set of stimuli, the AIS can activate antibody independent mechanisms like the T cell cytotoxic activity that have proven to be key in fighting viruses like HIV, cancer and other intracellular pathogens. Similarly, there are several cell-mediated immune mechanisms that have different functions and capabilities that will have implications on how effective the immune response and protection would be. These adaptive mechanisms are more difficult to measure but are increasingly being used as part of the strategies for treatments and prevention.

In summary, the induction of the appropriate combination of defence mechanisms is what ultimately decides how effective and protective the immune response is going to be. In that regard, depending on the status of activation of the immune system in general, the set of DAMPs that are present and the context in which they are encountered, the IIS would be activated and provide to the AIS a particular set of signals. Likewise, the set of signals received by the AIS will define the kind of mechanism that would be activated, the outcome of the immune response and ultimately the memory and protective immunity against a given pathogen. Hence controlling the context of the encounter of pathogens and the first steps of immune system activation is relevant to generating a robust, long-lasting and very effective immune response.

To underline how important this is, it is worth mentioning that many pathogens have evolved sophisticated mechanisms to control the early stages of immune system activation to increase the likelihood of establishing the infection and perpetuating themselves in the body. These mechanisms include, among others, hiding inside the cells, down-regulation of DAMPs, secreting decoy molecules and sending "wrong" signals to divert the immune system to ineffective pathways.

Altogether this reinforces the notion that the initial conditions of the system will ultimately decide the final results, thus providing a suitable triggering environment which will have a major impact on promoting beneficial pathophysiological outcomes (protective immune response, lessening undesired reactions or symptoms and recovery) from infectious challenges.

The present invention makes use of the finding that ultra-dilutions can be used to deliver a comprehensive and multilevel set of signals for modulating the initial conditions of the system toward an appropriate environment favouring beneficial outcomes from a challenge. In that way, by providing this environment, the formulation of the present invention can prepare the immune system for potential contacts with a given pathogen and therefore be used as a prevention tool. In the following, the formulation of the invention containing substances S_{A} and S_{B} in ultradiluted form is referred to as prophylactic UD (or PUD).

In the context of the present invention, it has been found that the formulation of the present invention can likely act at the very early steps of immune system activation to subtly prime the IIS but also to modulate the context of the interaction with the pathogen. Considering that the NP and CD in UD can deliver multiple signals carrying relevant information from the diluted substances and that they can be sensed by biological systems, an appropriate set of information can be delivered to provide an appropriate environment for pathogen encounter leading to a successful immune response. Depending on the dilution degree (concentration of molecules) PUD cannot contain direct physical signals to initiate an immune response by itself but can more broadly set the conditions to activate the immune system. In that case, the adaptive defence mechanisms such as antibody production and other cell-mediated mechanisms are activated in treated individuals as a result of, and only after, natural contact with the circulating pathogen. However, if the signals directed to modulate the environment are also accompanied with the presence of physical biomolecules (at ultra-low doses) like DAMPs or antigens, then a direct induction of an immune response can be achieved in a more effective way.

This stage of preparedness can be induced by using appropriate UD substances to trigger homeostatic mechanisms that promote favourable electrodynamic, biochemical and metabolic processes within the body and at cellular levels that can include but not be limited to modifications of water structure, ATP levels (increased), enzymatic activity, ligand receptor affinities, stability of protein-protein interaction, membrane potential and polarization status, regulation mechanisms, nucleic acid and chromatin structure (for expression of required genes), synthesis pathways, structure of actin filaments, cell proliferation and migration and many other mechanisms. On the other hand, pathogen ultra-dilutions can, to some extent, provide signals that mimic the interaction of the pathogen with the immune system.

By priming the appropriated homeostatic mechanisms, the effect of the PUD is mostly to increase the speed and the quality of the immune response rather than the levels (quantity) of the effector mechanisms. However, it can also have an impact on lowering the probability of developing symptoms of the disease in infected individuals and that those symptoms will be very mild if not entirely absent during infection.

By considering the body as a complex adaptative system and all the homeostatic mechanisms related or directly involved in the immune response, the present invention proposes the use of UD formulations as a highly effective preventive tool and a powerful approach for the control of epidemics. Prophylactic ultra-dilutions (PUD) can reduce susceptibility or increase resistance to the pathogen, and therefore can effectively provide protection leading to a significant and long-lasting reduction of the incidence of the disease within the treated population.

According to a preferred embodiment of the present invention, the pathogen is selected from the group consisting of bacteria, virus, yeast, fungi and protozoa.

In particular, each of core components A and B contain two or more substances S_{A1}, S_{A2}, ... and S_{B1}, S_{B2}, ... respectively, the maximum concentration of any of the substances being lower than 10⁻⁶ g/mL, preferably lower than 10⁻¹² g/mL, more preferably lower than 10⁻²⁴ g/mL

According to a preferred embodiment, the formulation comprises microquantities, nanoparticles or water coherent domains from the substances S_{A} and S_{B}. In this regard, it is particularly preferred that the microquantities, nanoparticles or water coherent domains are obtained by a process of agitated serial dilution or ultra-dilution.

As will be discussed in the context of the specific working example, it is further preferred that the core components A and B, and in particular the at least one substance S_{A} and S_{B} of the core components A and B, respectively, are contained in the formulation in equal amounts.

According to a particularly preferred embodiment and as will be discussed in further detail below, the disease to be treated is COVID19, the core component A containing biological material obtained from inactivated SARS-CoV-2 virus and the core component B containing at least one substance selected from the group consisting of cucurbitacins, in particular bryonioside, flavonoid glycosides, in particular saponarin, and diazanaphthalenes, in particular vasicinone and vasicine, as well as mixtures thereof. In this regard, it is further preferred that core component A further contains biological material obtained from inactivated *Streptococcus pneumoniae* bacteria, taking into consideration that COVID19 fatalities, respiratory failure from severe pneumonia caused either by SARS-CoV-2 itself or as a result of a secondary bacterial infection is the more frequent cause of death.

The selection of the substances S_{A1}, S_{A2}, ... and S_{B1}, S_{B2}, ... for the core components A and B respectively is based on a comprehensive analysis of systematically collected data. The systematic collection of data relates to collecting and classifying information related to the target disease and the causative agent. The source of this information is basically published information, preferably from published scientific papers that could be related directly to the pathogen and the disease, but also to any other microorganisms that share important characteristics. Official reports from governments, international organisations and institutions could also be considered including reports from the WHO, CDC, national or international institutions who deal with epidemics, and the like. Data mining tools and online resources are used as well.

The data search is focused on gathering all possible pieces of information that could lead to identification of the natural homeostatic mechanisms underlying the interaction of the infectious pathogen with the human body. All reactions, at macro, micro, cellular and molecular level will be considered as the expression of beneficial mechanisms. In that regard, depending on the intensity, harmful overreactions would be considered as homeostatic mechanisms functioning in an unbalanced or unregulated manner that could be potentially beneficial if modulated. Downregulated or suppressed reactions will also be considered with the aim of restoring them. The data collected will include but is not limited to: nature and classification of the pathogen (aetiology), structure, pathogenesis, epidemiology, diagnosis, symptoms, clinical observations, case reports, medical imagery, characteristics of the disease progression, markers of prognosis, risk factors, influence of pre-existing conditions, immune response, haematology parameters, sequels, reactions of other body systems, and any other relevant data.

All evidence will be used to build up a data base specific to targeted pathogens and later used for product design. Especially for clinical evidence, every reasonable attempt to collect as much information and from the largest number of individuals as possible will be made. With regard to the data analysis to identify patterns and principal features, all evidence collected will be classified as observations or confirmed clinical evidence. Further classifications will be made in evidence from the causative agent itself or related to the disease symptoms/reactions they cause (clinical data) as this is relevant for the designing the two main cores of the final product as described later.

Clinical data will be ranked according to their intensity, observed frequency and likelihood of occurring. Top ranking evidence will be considered as principal features and be more weighted for the selections of components. A close surveillance of new evidence is kept as the initial picture could change over time as the numbers increase and the results from research are being published.

The analysis will also be complemented and confirmed with what is known about the mechanisms involved in the pathogenesis of the disease, the physiological pathways and the immune response.

As outlined before, considering that the symptoms and reactions observed in disease are the result of the activation of homeostatic natural mechanisms for recovery and protection, a combination of substances from different origins that altogether induce similar mechanisms (effects) can be used in ultra-dilutions to subtly and safely prepare the organism to deal with the causative agent when encountered.

Components are selected based on careful analysis of gathered data and the rankings of the clinical evidence. The selection method for core A components are more specifically selected on the consideration that the biological microorganisms that cause the disease would provide the more complete set of signals to stimulate the natural homeostasis response. The aim of core component B is to assist on providing the best possible environment for core component A by inducing/priming a broader range of underlying homeostatic mechanisms that are not directly related to the pathogen but are needed to modulate the environment into appropriate (regulated or balance) pathways. The synergistic action of both cores then conditions the system for beneficial outcomes. As a rule, both core components A and B are safe.

Each core can have more than one if not several substances. According to a particularly preferred embodiment, equal parts of both core components will be mixed in the final formulation.

The at least one substance of core component A is of biological origin and specific to the infectious pathogens identified as the main cause of the target disease. It can contain more than one strain or isolates from the same species. The criteria for strain selection are supported by the aetiology of the disease, epidemiology data on circulating strains and the availability of useful isolates. Preferably the most recently available isolates representing the major and most frequent circulating strains will be used. In cases where the specific strains causing the disease are not available or not accessible, closely related isolates that resemble the main characteristics can be used.

The preferred source for core component A are strains or isolates of infectious microorganisms including viruses, bacteria, parasites and mycoplasma. In addition, if in the progression of a given disease subsequent infection with more than one pathogen could be involved, then the core component A can include those pathogens in addition to the one causing the primary infection. This refers to diseases where after a primary infection, complications could arise due to secondary infections with opportunistic pathogens. As an example, Flu infection caused by influenza virus in some circumstances could progress to pneumonia caused by other opportunistic pathogens like Streptococcus pneumoniae. In that case both influenza virus and other pathogens potentially causing complications are preferably contained in core component A.

A condition for the use of infectious pathogens is that, regardless of the source, all biological material must be of a high quality and safe, posing no risk by including it in the core component A. To ensure that, only fully identified, documented, highly pure and inactivated microorganisms are used. That implies that, as a rule, the infectious material is previously handled by a certified lab and that all possible contaminants from the host is removed down to the minimum possible level.

However, in some circumstances where the infectious microorganisms are not available, pathogen derived molecules can be used. In this case, it is preferable to use molecules derived from strains selected as per the criteria mentioned before. Although mixtures of individual molecules have a limited representation of the whole microorganisms, at least the main antigenic determinants of the pathogen can nonetheless be covered. Thus, more than one molecule can be used to have the wider spectrum of antigens as possible.

The source of those molecules can be pathogen cultures, recombinant technology, phage display technology, enzymatic digestion, molecules expressed in other microorganisms of cellular system or by synthesis. Any generally accepted methods able to render highly purified molecules can be used to produce them. Useful material could include pathogen derived or related proteins, peptides, polysaccharides, lipids, fragments and combination hereof. This can also be extended to the use of more complex structures like molecular complexes, membranes and derived structures.

In the case of molecules, using mixtures of molecules or complex structures, the selection and use is also decided in terms of purity, origin, identification, antigenicity and any other relevant characteristics as appropriate. As isolated components from microorganisms, they are significantly safer as the risks are minimal and the main safety concern is the presence of contaminants from the process of preparation.

Component selection for core component B is focused on the underlying homeostatic mechanisms of the disease. Those mechanisms can be stimulated by substances or combinations whose effects resemble the major pathophysiological reactions or reactions observed in the target disease, even when they can be not directly associated with the pathogen.

Accordingly, substances contained in core component B have general effects that better match the top-ranking clinical evidence from the data base of disease-related clinical observations. Core component B preferably contains equal parts of ultra-dilutions of more than one substance thus resulting in a mixture of different components. As described before, the safety of the core is paramount and so any possible dangerous effects will be avoided either by excluding the substances that may cause it or eliminating the risk by an appropriate method.

The at least one substance of core component B preferably includes dilutions of naturally occurring substances of any nature from plants, animals, mineral, synthetic or chemical origin. Those substances can be complex mixtures derived from raw extracts or purified molecules. Extracts can be semi purified, fractions from the purification or clarification process, deconvoluted mixtures, and can contain families of molecules and different isoforms of the same molecules. Molecules contained in the raw material can be proteins, polysaccharides, glycoproteins, metabolites, peptides lipids, alkaloids and fragments hereof.

Minerals can include metals, non-metals and derived salts. Chemically derived molecules can include simple inorganic and more complex organic molecules whether they are synthetic or natural occurring substances.

As cell dependent entities, viruses are generally cultured in cellular cultures of viral permissive cells although in some circumstances they could isolated directly from positive clinical samples. Biological raw material containing whole viral particles, disaggregated viral component, viral proteins and parts thereof, could be obtained from cultures of virus infected cells. The conditions for culturing will specifically depend on the virus and the cells that allow viral infection and replication. Usually immortalised cells lines are more likely to be used but primary cells could be also useful. Viruses can be present either in the cellular compartment or as secreted particles in the culture supernatant. Generally, culture samples containing viral material are purified to eliminate any other component from the cells or the culture media. Usually differential centrifugation is used to separate viral components from cellular derivatives.

Regardless of the method for obtaining the raw material and its composition, the concentration of viral components in the raw material after purification is determined and adjusted, preferably in terms of protein concentration or number of Plaque Forming Units as applicable. However, other detection methods could be used as well when necessary.

After obtaining the adjusted (standardised) raw material, it must be rendered no longer infectious by any inactivation method with a proven effectiveness to inactivate the virus. The inactivation procedure can include ultraviolet light irradiation, chemical treatment, heating or autoclaving. Chemical treatment is avoided if possible, to avoid introducing chemicals or derivatives of chemical reactions in the sample as they will be contaminants difficult to eliminate. Autoclaving at 133°C and 3 bars (300 KPa or 3 Atmospheres) of steam pressure for 20 min is so far the more effective method for inactivation and sterilisation, although very aggressive for the sample. Preferably, less drastic conditions for autoclaving (e.g. 121°C, 1 Atmosphere for 20-30 min) are used, in other to preserve the main constituent of the samples while rendering it no longer infectious.

The inactivation method to use should be validated although the inactivity of the product must be confirmed in an appropriate model, usually the same cellular culture used for viral replication. Only negative results (undetectable) from such models are accepted for the raw material to be noninfectious and safe to proceed to ultra-dilutions.

Preferably, the final sample is also sterile so it could comply with the test for sterility referred in applicable pharmacopoeias. If the method used for inactivation does not render a sterile sample, then the sample should be sterilised by a validated method. Sterilisation methods can include autoclaving or sterilising filtration (0.22 mm) under aseptic conditions.

The final biological material is tested for quality control using available methods.

Biological material derived from virulent bacteria is obtained from cultures in growth media. The composition of the culture media and the incubation conditions is specific to each bacteria species. Bacteria are cultured in either liquid or semi-solid media, but liquid media is preferred. Cultures are harvested at the exponential (logarithmic) phase of the growth curve to ensure a fresh and viable culture is used for the raw material. The number of bacteria per millilitre of media are quantified using available methods including turbidity, colony counting in semi-solid media or any other established method.

After culture, bacteria can be harvested by centrifugation and re-suspension of bacterial pellet in an appropriate buffer. Preferably, the culture media is completely removed by several steps of centrifugation, elimination of the supernatant and re-suspension of the pellet in buffer (washes). The media free bacteria suspension can be adjusted to a specific concentration. The final concentration of the raw material is decided on case by case basis, but typically about 10⁶ bacteria of colony forming unit per millilitre is preferable. The concentration of the final suspension can be verified using any of the method mentioned above.

Bacterial material should also be inactivated after obtaining the adjusted media free bacterial suspension. In this case heat or autoclaving inactivation methods are preferred. However, any other available method that is validated for inactivating a particular bacterium strain could be used.

Autoclaving at 133°C and 3 bars (300 KPa or 3 Atmospheres) of steam pressure for 20 min, is so far the more effective method for inactivation and sterilisation although very aggressive for the sample. Heating at normal pressure at 80-100oC for 1 hour is also an effective method for inactivation that could be used if it is validated for that purpose with a given bacteria strain. If any other validated and more gentle method for inactivation is available, it would be preferable to use including less drastic conditions for autoclaving (e.g. 121°C, 1 Atmosphere for 20-30 min) in other to preserve the main antigenic constituent of the samples while rendering it no infectious.

The inactivation method to use should be validated although the inactivity of the product must be confirmed in an appropriate test. For instance, culturing in bacteria specific semi-solid media where no colonies must growth after a reasonable culturing time. The test for confirming the inactivity of the material should provide the optimal growing conditions for that strain of microorganism.

Preferably, the final bacterial material is also sterile so it could comply with the test for sterility referred to in applicable pharmacopoeias. If the method used for inactivation does not render a sterile sample, then the sample should be sterilised by a validated method. Sterilisation methods could include autoclaving or sterilising filtration (0.22 mm) under aseptic conditions.

Only after the bacterial material is confirmed as noninfectious or sterile should it be used for preparing ultra-dilutions. The final biological material would be tested for quality control using available methods.

As mentioned above, the present invention also relates to a process for preparing the formulation disclosed above. The process comprises the step of
a) providing a starting solution A' and a starting solution B', on which core components A and B are based, said starting solutions containing substances S_{A} and S_{B}, respectively, at an initial concentration cᵢ, and
b) serially diluting the starting solutions A' and B' in multiple dilution steps, the dilution factor in the dilution steps being in the range from 2 to 1000.

Ultra-dilution from the raw material from both core component A and B will be prepared using the same methods to ensure optimal generation of NP and CD. The process of ultra-dilution consists in a serial dilution process which includes vigorous agitation between each step, so called agitated dilutions.

The dilution (deconcentrating) degree would be decided based on the nature of the raw material and its purpose (function) in the final formulation. Substances included in core A and B could have different degrees of dilution and each individual substance within any of the cores could be diluted to a different degree.

Preferably, the dilution factor in the dilution steps is 2, 5, 10, 100 or 1000.

It is further preferred that the total dilution factor by which the substances SA and S_{B} contained in the starting solutions at concentration cᵢ are diluted to obtain the final concentration is in a range from 10⁴ to 10⁴⁰⁰. The maximum concentration of physical molecules from the raw material in the final product could be in the range of 10⁻⁶ (ug, micrograms) to 10⁻¹⁵ (fg, femtograms) g/mL, as mentioned above.

As will be illustrated by way of the specific working example, it can be further preferred that for core component A and/or core component B multiple starting solutions A'₁, A'₂, ... and/or B'₁, B'₂, ... are provided in step a), and that step b) comprises the sub-step of combining the solutions for core component A and/or core component B after a number of dilution steps, in particular after the third dilution step.

The dilution process could be carried out manually or automatically using adequate equipment. All dilution steps except those involved in scaling up will be performed in closed glass vessels (recipients). Preferably, the solution is agitated in the dilution steps, as mentioned above. Thereby, a total minimum kinetic energy of 400 Joules/mL (Nm, Newton metro) in the form of impacts, shock waves, vibrations or combinations of circular motion at more than 1000 rpm (revolutions per minute) and vibration is supplied to the solution. The devices used for that agitation could include vertical shakers, vortex and acoustic or shock wave homogenisers.

The dilution process is preferably performed without the influence of strong magnetic fields so appropriate isolation or shielding should be in place if necessary. Weak magnetic waves of a frequency between 3 and 60 Hz are preferably present in at least a part of the dilution steps, as they can help stabilise the NP and CD in ultra-dilutions.

In the process, a mixture is preferably as a vehicle solvent, said mixture containing pure water or an aqueous solution selected from the group consisting of buffered isotonic solutions, salt isotonic solutions (up to 0.9% of the salt in water) or an hydroalcoholic solution, and ethanol, the amount of ethanol in the mixture being from 10 to 90% measured either by volume or weight.

The dilution process would be carried out under GMP conditions and a clean environment with minimal exposure to the external environment. Initial dilution steps, up to the 1012 deconcentrating factor, should be performed under aseptic conditions to keep the resulting dilution sterile.

The final formulation of the product will contain equal quantities of cores A and B. Both cores would be mixed in equal parts (volume or weight) at the dilution step just before starting to scale up the final product.

The process of scaling up the final product could be carried out using the same methodology of preparing the dilutions. The process of ultra-dilution preparation is very scaleable, thus large volumes of the final product (bulk product) could be obtaining by doing the final dilution steps in an incremental volume. This feature of preparing formulations by sequential dilutions, makes the scale up process very easy and quick and allows to go from formulation design to mass production of final product in a very feasible and cost-effective way.

Once the cores A and B are formulated together, they would be further diluted as mix until the final dilutions of each substances are achieved. In the case of using different dilution factors for each substance in the final formulations, the individual components of each core would be prepared in a way that after formulation they all can be diluted together to their final dilutions in a 3-6 subsequent steps. Typically, the scale up would start between the last 3-5 dilution steps depending on the dilution factor. All the conditions described in the ultra-dilution preparation method would be observed, but would consider the mix as a simple substance, thus the dilution process remains the same. The scale up would be done using the appropriate vehicle for the oral delivered product which preferably would be the an ethanolic aqueous solution.

The final bulk product would be serially diluted substances from both core A and B at corresponding dilution degrees or concentrations. The final product would be orally delivered thus it must comply with the regulations for this kind of product. It could contain up to 50% but not lower than 10% ethanol in the vehicle. The function of the ethanol would be first to increase stability of the NP and CD (so the information they carry) and second as a preservative.

Depending on the formulation and its applications, the presentation of the product could be either a liquid solution or compressed tablets. In the case of liquid solutions, the bulk final product could be dispensed directly into the delivering recipients using available filling technology. The recipients could be made of plastic or glass and could include a dispensing device.

The preparation of compressed tablets would involve an additional process of embedding the bulk liquid product into the tablets so would require another formulation step.

The platform also involves a strategy for using the final product for prophylaxis against targeted disease or pathogens in large populations. This strategy in general includes a specific treatment dosage and schedule, administration and distribution roadmap, data gathering, testing (to identify infected individuals), interactive interaction with individuals through an artificial intelligence powered application and follow up. This general strategy would be very flexible and adaptable to changing conditions and dependent on the target pathogen/disease and its epidemiology characteristics, urgency of the intervention and collected data.

As mentioned above, the present invention relates in particular to an orally delivered preventive formulation to specifically target and prevent disease symptoms relating to COVID-19. Specifically, a formulation is provided, which effectively and safely promotes beneficial pathophysiology and immune outcomes for individuals who are exposed to SARS-CoV-2.

Hence, the present invention relates to a formulation for the prophylactic treatment of COVID-19 comprising two core components, of which a first core component A contains biological material obtained from inactivated SARS-CoV-2 virus as substance S_{A}, and a second core component B contains at least one substance S_{B} selected from the group consisting of cucurbitacins, in particular bryonioside, flavonoid glycosides, in particular saponarin, and diazanaphthalenes, in particular vasicinone and vasicine, as well as mixtures thereof, each of the substances S_{A} and S_{B} being contained in the formulation in a concentration lower than 10⁻⁶ g/mL, preferably lower than 10⁻⁹ g/mL, more preferably lower than 10⁻¹² g/mL.

According to a particularly preferred embodiment, core component A further contains biological material obtained from inactivated *Streptococcus pneumoniae* bacteria and/or from influenza virus, as will be discussed in the context of the specific working examples.

The homeostatic mechanisms considered by the present invention in relation to COVID-19 are discussed in the following:
COVID-19 is caused by the infection with SARS-CoV-2. It is suggested the virus could get into the cells using the angiotensin-converting enzyme 2 (AC2) as a host receptor. The spike S protein of the virus could bind the enzymatic domain of AC2 and trigger endocytosis and translocation of the virus into endosomes.

AC2 is a Zinc containing, single-pass transmembrane enzyme involved in blood pressure control by catalysing the hydrolysis of angiotensin II into angiotensin. This enzyme It is expressed in the surface of many cells, including epithelial cells, vascular endothelium, smooth muscle, lungs (alveolar endothelium), heart, kidney, and intestines (enterocytes of the small intestine); which could potentially be infected by SARS-CoV-2. It is also predicted to express in the cerebral cortex, striatum, hypothalamus, and brainstem. It is not clear whether the interaction of the virus with AC2 affects the function of this protein on blood pressure control and if that promotes lung damage. However, the interaction of viral proteins with many other cell surface proteins have been reported from in vitro and in silico studies but still requires confirmation, and the biological implication of such interaction needs more investigation.

Infection of endothelial cells may allow the virus to pass from the respiratory tract to the blood and then across the blood-brain barrier into the brain. Once in the brain, replication of the virus may cause neurological disorders.

### Clinical observations

The symptoms vary from country to country. The mild symptoms present at the beginning of the disease appear to be common to all countries but with slight variation in frequency, appearance and intensity. They are also related to differences in the incubation period.

### Asymptomatic individuals

No symptoms have been systematically collected in asymptomatic individuals although very subtle symptoms should be present.
- Some data suggest that strokes could be a cause of death in asymptomatic individuals and thus suggesting some damage of the virus to the endovascular system.
- Slight opacification on the lung images have been reported in asymptomatic patients suggesting viral replication and some lung inflammation/injury.

### Mild Disease

Mild symptoms are present in around 81% of infected individuals. Symptoms are usually mild and begin gradually and are Flu like. There are common symptoms present in almost all sick individuals, but other less frequent reactions may be present.

| Common Symptoms | Other Symptoms |
|---|---|
| Tiredness | Aches and pains |
| Mild fever | Runny nose |
| Dry cough | Diarrhoea |
| Malaise | Muscle pain |
| Headache | Nasal congestion |
| | Sore throat |

### Moderate Disease

Depending on age, comorbidities, immunological status and other factors, symptoms in patients with mild disease could rapidly intensify and progress to moderate and severe. In addition to symptoms observed in mild disease, new ones appear, mostly related to the cardiovascular and respiratory system.

### Symptoms

- Cough
- Shortness of breath
- Tachypnea

### Severe Disease

The symptom pictures in severely ill patients get very complex and depend on how critical the individual is. However, most of the symptoms are related to damage from viral infection to the cardiovascular and respiratory system and immune system activation.

### Symptoms

- Fever can be absent or moderate
- Severe dyspnea
- Tachypnea (respiratory rate > 30/minute)
- Pneumonia
- Lung oedema
- Acute respiratory distress syndrome
- Respiratory failure - 50% lung infiltrates within 24 to 48 hours
- Lymphopenia
- Thrombocytopenia
- Cardiac injury
- Sepsis
- Septic shock
- Multiple organ dysfunction
- Stroke (also observed in asymptomatic individuals)

The case fatality rate for critical patients is 49%.

### Specific reactions of main systems

### Blood Disorders

- Increase in blood clotting disorders, mainly high blood coagulation activity
- Increase of the number of epithelial cells circulating in the blood
- Thrombocytopenia

### Cardiovascular system

Damage to the cardiovascular system and particularly to the heart could be caused by a direct viral infection in the heart, as a consequence of the immune response and the physiological, metabolic distress of the body (lack of oxygen, low blood pressure, inflammation, etc.). Cardiovascular symptoms could be present at early stages in mild disease.

### Symptoms

- Tachypnea (respiratory rate > 30/minute)
- Heart palpitations
- Chest tightness
- Low blood pressure
- Arrhythmia
- Myocarditis and myocardial injury
- Heart failure
- Stroke

### Immune Response

The uncontrolled or ineffective immune response is suggested to be the main cause of disease progression.
- Overactivation of the immune system when disease progresses to moderate to severe.
- Neutrophilia.
- Lymphopenia
- Thrombocytopenia
- Many inflammatory markers are produced at higher levels: C-reactive protein (CRP), interleukin (IL) 2, IL-7, IL-10, GCSF (granulocyte colony-stimulating fac- tor), IP10 (interferon gamma-induced protein 10), MCP1 (monocyte chemotactic protein 1), MIP1A (macrophage inflammatory protein alpha), and TNF- α (tumour necrosis factor-α).
- Coagulation cascade could be activated, probably because complement deposition in infected epithelial cells circulating in the blood.

### Nervous system

Neurological symptoms are observed in 36% of the patients and more frequent in severe patients (46-84%). The virus can cross the blood-brain barrier as it has been detected in the brain of death individuals. It is suggested that SARS- CoV2 could infect the olfactory neurons getting access to the brain through the olfactory nerve and could spread to the hypothalamus where it can affect the respiratory centre and cause respiratory distress and failure.

### Symptoms

- Loss of smell or taste
- Guillain-Barré syndrome
- Headache
- Dizziness
- Encephalitis
- Muscle weakness
- Seizure
- Hallucinations

In general, most of the symptoms and reaction occurring during SARS-CoV-2 infection are related to the inflammatory mechanisms of the immune system and the direct damage of the virus on infected cells. Depending on the localisation of this inflammation and cell damage, then the reactions could be generating different symptom pictures. In severe cases inflammatory reactions appears to be more systemic involving several organ systems. However, in any case, overexpressed inflammatory mechanisms are responsible for the appearance of visible symptoms and the degree of them are clearly linked to the ability of the body to control such reactions and direct them to the appropriate pathway. The success of using corticosteroids (glucocorticoids) that suppress inflammation and immunity and assist in the breakdown of fats, carbohydrates, and proteins in treating severe cases of the disease also supports the association of severe stages of the disease with an over inflammatory reaction of the body. In that sense, asymptomatic infections could be considered as a natural course of the infection in individuals where homeostatic inflammatory mechanisms occur in a modulated manner. Thus, contact with the virus or its components in a controlled inflammatory environment together with a proper regulation of the direct virus damage becomes a key for leading to beneficial outcomes. This should be the focus of the approaches to control the epidemic and that is precisely the basis of the present invention.

In addition, and supporting the above idea, there are several risks factors that have been associated with a bad prognosis of the disease. Among them, age, comorbidities and opportunistic infections are the main risk factor for progression to severe stages of the disease and have been clearly linked to fatalities. Patients without comorbidities have a lowercase fatality rate (0.9%).

Age and comorbidities are closely linked, as the elderly over 60 year is the age group at higher risk which is at the same time the group that shows higher incidence of underlying chronic medical conditions. The background comorbidities that has been identified as important risk factors include:
- Cancer
- Chronic kidney disease
- COPD (chronic obstructive pulmonary disease)
- Immunocompromised state
- Obesity
- Serious heart conditions, such as heart failure, coronary artery disease, or cardiomyopathies
- Sickle cell disease
- Type 2 diabetes mellitus

Many of these conditions are related to unbalanced health stages that compromise the immune system.

On the other hand, secondary opportunistic infections leading to pneumonia have been associated with around 15% of hospital cases and around 50% of COVID-19 fatalities presented with some bacterial infections that could have contributed to their death. The secondary infection is facilitated by the direct damage from SARS-CoV-2 and the malfunction of the immune system. In COVID 19 fatalities, respiratory failure from severe pneumonia caused either by SARS-CoV-2 itself or as a result of a secondary bacterial infection is the more frequent cause of death. In contrast, the rate of secondary infections is significantly lower on survivors. The avoidance of secondary infections has been one of the main targets of the treatments recommended for COVID19 patients and the prophylactic use of antibiotics has dramatically reduced the impact of opportunistic bacterial infections. However, secondary bacterial infections should also be considered in preventive alternatives.

The more frequent opportunistic bacteria related to severe cases of COVID-19 are: *Staphylococcus aureus, Streptococcus pneumoniae* and *Haemophilus influenzae.* However, other bacteria strains have been reported depending on the region, diagnostic tools and clinical management.

In addition, they are some other pathogens unrelated to SARS-CoV-2 generating similar symptomatology at some stage of the disease. A clear example is that COVID19 disease closely resemble the disease caused by Influenza virus infection. This suggests that at some points both viruses share common homeostatic reactions although the immune response endpoints could be different for each of them. This is another factor that should be considered, as there is a potential to use related pathogens to prime appropriate homeostatic mechanisms. This approach is novel and has never been considered.

According to a specific and preferred embodiment, the present invention relates to a formulation for the prophylactic treatment of COVID19 disease.

### Core component A

The components included in core component A are chosen based on the infectious pathogens that are the direct cause or are related to COVID19 disease.

Specifically, at least one of the strains presented in the Table 1 from each pathogen is included in core component A.

### Core component B

The at least one substance contained in core component B consist in molecules from plant and chemical origin that are selected to target the underlying mechanisms from the more frequent symptoms observed in mild disease, but also considering the more serious reactions observed in moderate and severe stages.

Specifically, core component B contains at least one substance selected from the group given in Table 2. More preferably, least one molecule from each compound family and source is contained in core component B. Having the possibility of using a set of molecules that share some redundancy and balance in terms of mechanisms, increases the probability of inducing an effect in a wide spectrum of body environments (initial conditions).

**Table 1; substances of core component A**

| Origin | | Rationale | Source | Raw material preparation |
|---|---|---|---|---|
| Pathogen | Strains/Serotype/Lineage | | | |
| SARS-CoV-2 | Spike D614G Mutant | Causative pathogen of COVID19. | *In vitro* culture of infected Vero cells | Purified culture supernatant is adjusted at given concentration and inactivated by 1h UVC exposure following by sterilising filtration (0.22 um) or sterilized at 121°C, 1 Atmosphere for 30 min |
| *Streptococcus pneumoniae* | Strains from serotypes 3, 6A or 6B, 14, 1, 4, 9, 8, 19F, 23F and 7F | Pathogen frequently causing secondary opportunistic infection during COVID19 leading to complications, pneumonia and death. | Bacteria cultures in blood containing media in 5% carbon dioxide | Suspension of bacteria free of culture media is adjusted at a given concentration and autoclaved at 133°C and 3 Atmospheres for 20 min. |
| *Influenza virus* | AH1N1, AH3N2, AH5N1, AH7N9, AH9N2 and B | Related pathogen causing reactions derived from the activation of similar homeostatic mechanisms of those of COVID19. | Allantoic fluid of infected embryonated chicken egg or in vitro cultured of infected MDCK cells | Purified culture supernatant is adjusted at given concentration and inactivated by 1h UVC exposure following by sterilising filtration (0.22 um) or sterilized at 121°C, 1 Atmosphere for 30 min |

**Table 2; substance of core component B**

| Molecule | Nature | Formula | Effects/Mechanisms | Rationale | Source |
|---|---|---|---|---|---|
| Bryonioside | Glycoside, Cucurbitacins | 7-hydroxy-1-(5-hydroxy-6-methyl-6-{[3,4,5-trihydroxy-6-(hydroxymethyl )oxan-2-yl]oxy}heptan-2-yl)-3a,6,6,9b,11a-pentamethyl-1H,2H,3H,3aH, 3bH,4H,6H,7H, 8H,9H,9aH,9bH 10H,11H,11aH-cyclopenta[a]ph enanthren-10-one | - Anti-viral | Inhibits viral replication and promote cell defence mechanisms | Standardised extract from Bryonia Alba, Bryonia dioica (White bryony or Wild hop). Plant from the family of Cucurbitacea e |
| | | | - Antiinflammatory | | |
| | | | - Inhibiting protein synthesis at ribosome level. | | |
| Saponarin | Flavonoid glycosides | Isovitexin-7-O-beta-D-glucopyranoside | - Increase cell viability by membrane stabilizing activity | Decrease damage at cellular level due to oxidative burst. | |
| | | | - Hepatoprotection | | |
| | | | - Inhibit oxidant enzymes like: Cytochromes P450, xanthine oxidase. | Glucose level control helps to stabilise metabolic distress and minimise the riskin diabetes patients. | |
| | | | - Reduce blood glucose levels | | |
| | | | - Increase reduced glutathione (GSH). Reduce lactate dehydrogenase (LDH). | Reducing inflammation at local level. | |
| | | | - Activate AMP-activated protein kinase. | Reducing fever due to the release of pyretic mediators | |
| | | | - Inhibition of NF-*κ*B, ERK, and p38 signalling (antiinflammatory). | | |
| Vasicinone | Diazanaphthalenes | 1H-Pyrrolo[2,1-b]quinazolin-9-one, 3-hydroxy-2,3-dihydro-3-hydroxy-2,3-dihydropyrrolo[ 2,1-b]quinazolin-9(1H)-one | - Bronchodilator | Helps with | Standardised extract from Justicia adhatoda. Plant from the family of Acanthaceae |
| | | | - Antibacterial | respiratory | |
| | | | - Assist with respiratory disorders including cough, colds, asthma bronchial catarrh, bronchitis, and tuberculosis. | distress Cough Anti bacterial activity helps in preventing secondary infections | |
| Vasicine | Diazanaphthalenes | 1,2,3,9-tetrahydropyrrol o[2,1-b]quinazolin-3-ol | - Bronchodilator | Helps with respiratory distress | |
| | | | - Antiinflammatory | | |
| | | | - Cholinesterase inhibitor | Reduces inflammation | |

### Preparation of components

The raw materials for core component A and the source materials of core component B were used as the starting materials for preparing the ultradilutions to achieve the final formulation of the present invention.

For core component A, the more recent isolates of the listed strains were obtained as inactivated material as described. All strains were diluted in ethanol:water (30%) solution two times with a 1:100 dilution factor. The resulting dilutions were mixed by pathogen, diluted once more and then all pathogens mixed in equal proportions for continuing the dilutions steps.

In the case of core component B, the extracts from the plants were tested for the presence of the listed molecules in the appropriate proportions. In some cases, specific molecules were acquired as individual components from different manufacturers and added to the extract to get equal proportions with the other molecules. Each of the source materials were diluted 1:100 in ethanol:water (30%) two times, and then the resulting dilutions mixed in equal proportions for continuing the dilutions steps.

The dilution factor for each serial dilution was 100 and the number of dilution steps were defined individually for each material. The method for performing the ultra-dilutions was previously described.

Core components A and B were processed independently until the 4th to last dilution. At this dilution, equal parts of both cores were mixed and diluted 3 times more under the same conditions but scaling the volume up so to produce large quantities of the final bulk product.

The entire process was carried out under GMP conditions and a strict quality assurance system.

Samples of the bulk product were submitted to a rigorous quality control testing to confirm that the main properties and characteristic defined in the specification of the final product were within the acceptable limits.

The bulk product was dispensed into the final recipient under GMP conditions. The final presentation of the product is an oral administered formulation in a multidose glass bottle.

The final product could be also presented as individual dose disposable plastic container. In this case, polypropylene plastic ampules are filled with the formulation of the present invention using a Blow Fill Seal (BFS) technology.

In both cases the final product is ready to use and does not require any manipulation apart from dispensing the dose treatment directly in the mouth of the individual.

The formulation of the present invention is designed to gently prime the homeostatic mechanisms that provide a favorable environment for developing an appropriate immune response after natural contact. The action is intended to occur in several body systems and particularly the immune system but considering the interaction of all systems.

The formulation of the present invention can be used as prevention alternative for SARS-CoV-2 infection, and so its uses are related to all potential applications for decreasing the incidence and increase protection to COVID19.

The dose consists of 200 uL of the final product administered orally, and this dose remain the same regardless of the frequency of administration. The schedule of administration depends on the exposure risk of the target populations and is decided according to a risk matrix.

## Claims

1. Formulation for the prophylactic treatment of an infectious disease comprising two core components, of which a first core component A contains at least one substance S_{A} related to the target disease, and a second core component B contains at least one substance S_{B} for targeting a disease-related homeostatic mechanism that assists core component A in inducing a beneficial environment for developing appropriate protection mechanisms against the disease, wherein substance S_{A} is a biological material from an inactivated pathogen or a part thereof, and substance S_{B} is a synthetic or naturally occurring substance different to substance A, each of the substances S_{A} and S_{B} being contained in the formulation in a concentration lower than 10⁻⁶ g/mL.

2. Formulation according to claim 1, wherein the pathogen is selected from the group consisting of bacteria, virus, yeast, fungi and protozoa.

3. Formulation according to claim 1 or 2, wherein the disease to be treated is COVID-19, the core component A containing biological material obtained from inactivated SARS-CoV-2 virus and the core component B containing at least one substance selected from the group consisting of cucurbitacins, in particular bryonioside, flavonoid glycosides, in particular saponarin, and diazanaphthalenes, in particular vasicinone and vasicine, as well as mixtures thereof.

4. Formulation according to claim 3, wherein core component A further contains biological material obtained from inactivated *Streptococcus pneumoniae* bacteria and/or from influenza virus.

5. Formulation according to any of the preceding claims, wherein each of core components A and B contain two or more substances S_{A1}, S_{A2}, ... and S_{B1}, S_{B2}, ... respectively, the concentration of any of the substances being lower than 10⁻⁶ g/mL, preferably lower than 10⁻¹² g/mL, more preferably lower than 10⁻²⁴ g/mL.

6. Formulation according to any of the preceding claims, wherein it comprises microquantities, nanoparticles or water coherent domains from the substances S_{A} and S_{B}.

7. Formulation according to claim 6, wherein the microquantities, nanoparticles or water coherent domains are obtained by a process of agitated serial dilution or ultra-dilution.

8. Formulation according any of the preceding claims, wherein the core components A and B, and in particular the at least one substance S_{A} and S_{B} of the core components A and B, respectively, are contained in the formulation in equal amounts.

9. Process for preparing the formulation according to any of claims 1 to 8, comprising the step of
a) providing a starting solution A' and a starting solution B', on which core components A and B are based, said starting solutions containing substances S_{A} and S_{B}, respectively, at an initial concentration cᵢ, and
b) serially diluting the starting solutions A' and B' in multiple dilution steps, the dilution factor in the dilution steps being in the range from 2 to 1000.

10. Process according to claim 9, wherein the dilution factor in the dilution steps is 2, 5, 10, 100 or 1000.

11. Process according to claim 9 or 10, wherein the total dilution factor by which the substances S_{A} and S_{B} contained in the starting solutions at concentration cᵢ are diluted to obtain the final concentration is in a range from 10⁴ to 10⁴⁰⁰.

12. Process according to any of claims 9 to 11, wherein for core component A and/or core component B multiple starting solutions A'₁, A'₂, ... and/or B'₁, B'₂, ... are provided in step a), and step b) comprises the sub-step of combining the solutions for core component A and/or core component B after a number of dilution steps, preferably after the third dilution step.

13. Process according to any of claims 9 to 12, wherein in the dilution steps the solution is agitated, whereby a total minimum kinetic energy of 400 Joules/mL (Nm, Newton metro) in the form of impacts, shock waves, vibrations or combinations of circular motion at more than 1000 rpm (revolutions per minute) and vibration is supplied to the solution.

14. Process according to any of claims 9 to 13, wherein in at least a part of the dilution steps the solution is under the influence of magnetic waves of a frequency between 3 and 60 Hz.

15. Process according to any of claims 9 to 14, wherein a mixture is used as a vehicle solvent, said mixture containing
pure water or an aqueous solution selected from the group consisting of buffered isotonic solutions, salt isotonic solutions, in particular containing up to 0.9% of the salt in water, or an hydroalcoholic solution, and
ethanol, the amount of ethanol in the mixture being from 10 to 90% measured either by volume or weight.
